# EUROPÄISCHE PATENTSCHRIFT

(11) **EP 2 837 363 B1**
(45) Veröffentlichungstag und Bekanntmachung des Hinweises auf die Patenterteilung: **30.09.2015**
(21) Anmeldenummer: 14175026.5
(22) Anmeldetag: 30.06.2014
(51) Int. Cl.: A61F 2/18

(54) **Stapesprothese mit Schnappverschluss**
Stapes prosthesis with snap-on fastening
Prothèse de stapédectomie dotée de fermeture à déclic

(30) Priorität: 08.08.2013 DE 102013108566
(43) Veröffentlichungstag der Anmeldung: 18.02.2015
(73) Patentinhaber: Heinz Kurz GmbH Medizintechnik, 72144 Dusslingen (DE)
(72) Erfinder: Eiber, Dr., Albrecht, 71384 Weinstadt (DE); Steinhardt, Uwe, 72145 Hirrlingen (DE); Kurz, Heinz, 72144 Dusslingen (DE)
(74) Vertreter: Kohler Schmid Möbus Patentanwälte

(56) Entgegenhaltungen:
- US-A- 4 292 693
- US-A1- 2006 241 755

## Beschreibung

Die Erfindung betrifft eine Gehörknöchelchenprothese, die zum Ersatz oder Überbrücken mindestens eines Elements der menschlichen Gehörknöchelchenkette ausgebildet ist, mit einem länglichen, Schall übertragenden Prothesenkörper, welcher an seinem einen Ende ein erstes Ankoppelelement aufweist, das zur mechanischen Verbindung mit dem Ambossfortsatz gestaltet ist und einen starr mit dem Prothesenkörper verbundenen Aufnahmeteil zur Aufnahme des freien Endabschnitts des Ambossfortsatzes sowie einen im Wesentlichen U-förmig gebogenen Bügelverschluss aufweist, welcher drehbar im Aufnahmeteil gelagert ist und zur Befestigung des Ambossfortsatzes über den im Aufnahmeteil aufgenommenen freien Endabschnitt des Ambossfortsatzes geschwenkt werden kann, wobei der Prothesenkörper an seinem anderen Ende ein zweites Ankoppelelement zur mechanischen Verbindung mit einem weiteren Glied oder Teilen eines Gliedes der Gehörknöchelchenkette oder direkt mit dem Innenohr aufweist.

Derartige in der Regel als Stapes-Prothesen ausgestaltete passive Gehörknöchelchenprothesen sind schon lange bekannt und beispielsweise in US-A 3,196,462, US-A 4,292,693 oder in US 2006/0241755 A1 beschrieben, wobei allerdings bei der Prothese gemäß US-A 3,196,462 der Bügelverschluss in nicht U-förmig, sondern eher O-förmig gebogenen ist.

Ähnliche Stapes-Prothesen, allerdings ohne die Henkeiförmige Konstruktion des Bügelverschlusses, sind aus US-A 3,711,869 oder US-A 3,931,648 bekannt.

Das menschliche Mittelohr mit seinen Gehörknöchelchen hat die Funktion, die über den äußeren Gehörgang auf das Trommelfell auftreffenden Schallwellen auf das mit Flüssigkeit gefüllte Innenohr zu übertragen. Die drei Gehörknöchelchen sind der am Trommelfell befestigte Hammer (lat. malleus), der Steigbügel (lat. stapes), der über seine Fußplatte (lat. basis stapedis) mit dem Innenohr verbunden ist, und der Amboss (lat. incus), der sich zwischen dem Hammer und dem Steigbügel befindet und mit diesen gelenkig verbunden ist. Beispielsweise die Otosklerose ist eine Erkrankung des menschlichen Felsenbeins (= Knochen, in dem das gesamte Ohr sitzt), bei der es durch entzündungsähnliche Knochenumbauprozesse zu einer Fixierung des normalerweise locker schwingenden Steigbügels kommen kann. Dadurch wird das Schallsignal nicht oder nur unvollkommen über die Gehörknöchelchenkette auf das Innenohr übertragen, was zur Schwerhörigkeit führt.

Gehörknöchelchenprothesen dienen der Verbesserung der Schallübertragung bei unterschiedlichen pathologischen Befunden. Sie werden verwendet, um bei ganz oder teilweise fehlenden oder geschädigten Gehörknöchelchen des menschlichen Mittelohrs den Schall vom Trommelfell zum Innenohr zu übertragen. Die Gehörknöchelchenprothese weist dabei zwei Enden auf, wobei je nach den konkreten Gegebenheiten das eine Ende der Gehörknöchelchenprothese am Trommelfell oder einem Gehörknöchelchen und das andere Ende der Gehörknöchelchenprothese beispielsweise am Steigbügel der menschlichen Gehörknöchelchenkette befestigt oder direkt ins Innenohr getaucht wird.

Drei besonders häufig verwendete Arten von Gehörknöchelchenprothesen sind die Steigbügel-Prothesen, die Partial-Prothesen und die Total-Prothesen. Steigbügel-Prothesen (=Stapes-Prothesen) werden in der Regel am langen Ambossfortsatz fixiert und ragen über einen Stempel (=Piston) ins Innenohr oder sitzen mit dem Stempel auf einem Gewebestück auf, welches das Innenohr abdichtet. Partial-Prothesen liegen meist mit einer Kopfplatte am Trommelfell an und stellen eine Verbindung zum Steigbügelköpfchen her. Total-Prothesen verbinden das Trommelfell mit der Steigbügel-Fußplatte.

Des Weiteren unterscheidet man generell zwischen *passiven* Gehörknöchelchenprothesen und *aktiven* Hör-Implantaten mit elektronischen Verstärkerelementen, die über ein externes Energieteil betrieben werden. Bei der eingangs definierten gattungsgemäßen Gehörknöchelchenprothese handelt es sich um eine passive Prothese, bei der am oberen Ende des Prothesenkörpers ein Aufnahmeteil vorgesehen ist, welches unter dem freien Endabschnitt des Ambossfortsatzes angelegt werden kann. Zur Befestigung der Prothese am Amboss wird dann ein beweglicher U-förmig gebogener Bügelverschluss über den Ambossfortsatz verschwenkt.

Bei allen im oben zitierten Stand der Technik beschriebenen derartigen Gehörknöchelchenprothesen (mit Ausnahme der Prothese gemäß US-A 4,292,693) ist jedoch keine Sicherung gegen ein ungewolltes selbsttätiges Abrutschen des Bügelverschlusses nach der Befestigung durch den Chirurgen im Mittelohr des Patienten vorgesehen. Der Bügel kann also ohne weiteres nach Abschluss der Implantation, insbesondere durch post-operative Narbenzüge während des Heilprozesses von selbst über den freien Endabschnitt des Ambossfortsatzes zurückschwenken. Danach ist es dann wahrscheinlich, dass auch die Auflage des Ambossfortsatzes auf dem Aufnahmeteil verrutscht und die Gehörknöchelchenprothese dann "frei in der Luft hängt", wobei die Schallleitende Verbindung zum Innenohr natürlich unterbrochen ist und der Erfolg der vorangegangenen Mittelohr-Operation zunichte gemacht wird.

Diesen erheblichen Nachteil versucht die Gehörknöchelchenprothese gemäß US-A 4,292,693 zu beheben, indem dort das Anbringen zweier gegenüberliegender Nocken an der zylinderförmigen Außenwand des starr mit dem Prothesenkörper verbundenen Aufnahmeteils vorgeschlagen wird. Die Nocken sollen unter einem Winkel zulaufende schräge Außenflächen aufweisen und in einer abrupt in Richtung auf das Aufnahmeteil verlaufenden Schulter enden. Diese Konstruktion soll es ermöglichen, den Henkelförmigen Bügelverschluss zwar beim Verschließen über die ansteigenden Außenflächen der Nocken (und dabei natürlich über den freien Endabschnitt des Ambossfortsatzes) zu ziehen, jedoch hinterher ein selbstständiges Zurückschwenken des Bügelverschlusses verhindern.

Nachteilig bei dieser bekannten Gehörknöchelchenprothese ist einerseits die ziemlich aufwändige Konstruktion, wobei man sich klarmachen muss, in welchen winzigen Dimensionen sich derartige Nocken an dem Aufnahmeteil sich bewegen müssten: Das Aufnahmeteil selbst wird in der Regel einen Durchmesser im Bereich zwischen einem halben und einem Millimeter aufweisen. Die - relativ kompliziert strukturierten - Nocken müssten demnach in einer Größenordnung von 100µm und darunter hergestellt werden, was fertigungstechnisch selbst heute im Zeitalter der Lasertechnik (also über drei Jahrzehnte nach Publikation der US-A 4,292,693) eine kaum zu bewältigende Herausforderung darstellt und jedenfalls nicht in nennenswerten Stückzahlen und zu einem konkurrenzfähigen Preis machbar ist.

Aus diesem Grund dürfte wohl der Vorschlag der US-A 4,292,693 bis heute in der Praxis nicht aufgegriffen worden sein, was zum Beispiel implizit auch aus der oben genannten US 2006/0241755 A1 hervorgeht, wo zwar der Stand der Technik gemäß US-A 4,292,693 einschließlich der oben geschilderten Rückhalte-Funktion mittels der Nocken ausführlich beschrieben und gewürdigt wird, für die eigene in der US 2006/0241755 A1 vorgeschlagene neue Erfindung jedoch gar nicht in Erwägung gezogen wird.

Ein weiterer gravierender Nachteil der "Nockenlösung" besteht in der Gefahr eines Abreißens des Bügelverschlusses beim Ziehen über die Nocken: Sind letztere nämlich mit ihren schrägen Außenflächen nur um einen geringen Winkel verdreht gegen die Zugrichtung des Bügels angeordnet - was fertigungstechnisch aufgrund der endlichen Toleranzen ohne Weiteres passieren kann - oder stehen die Nocken um ein Weniges zu weit aus dem Aufnahmeteil heraus, so wird sich in vielen Fällen der - äußerst zarte - Bügel an den Nocken verhaken und bei der Weiterbewegung einfach zerrissen werden. Die negativen Folgen für den Patienten, insbesondere wenn der Vorgang während der Implantation unbemerkt bleibt, können verheerend sein. Aufgrund der relativ beengten Verhältnisse und der nicht immer klaren Sicht, kann dies dazu führen, dass der Chirurg das Problem nicht erkennt, weiter versucht den Bügel über den Ambossfortsatz zu ziehen und dabei das Innenohr durch ein unkontrolliertes Eintauchen des Implantates irreparabel geschädigt wird.

Aufgabe der vorliegenden Erfindung ist es demgegenüber, eine gattungsgemäße passive Gehörknöchelchenprothese der eingangs beschriebenen Art mit möglichst simplen technischen Mitteln und wirtschaftlich preisgünstig dahin gehend zu verbessern, dass sie einerseits fertigungstechnisch besonders einfach und preiswert herzustellen ist, andererseits die oben geschilderten Gefahren bei Verwendung etwa einer Gehörknöchelchenprothese gemäß der oben zitierten US-A 4,292,693 sicher vermeidet, wobei sie aber dennoch einen guten Schutz gegen ein unbeabsichtigtes Zurückfahren des Bügelverschlusses über den Ambossfortsatz nach Beendigung der Implantation bietet.

Erfindungsgemäß wird diese relativ anspruchsvolle Aufgabe auf ebenso überraschend einfache wie wirkungsvolle Art und Weise dadurch gelöst, dass der Bügelverschluss mindestens eine Ausbuchtung aufweist, die auf das Aufnahmeteil gerichtet ist und so weit aus dem Bügelverschluss vorsteht, dass sie beim Verschwenken des Bügelverschlusses eine Außenfläche des Aufnahmeteils berührt.

Auf diese Weise kann einerseits sicher verhindert werden, dass der Bügelverschluss nach seinem Verschwenken über den freien Endabschnitt des Ambossfortsatzes selbsttätig wieder zurückschwenkt und dabei die Verbindung der Gehörknöchelchenprothese zum Ambossfortsatz löst. Anderseits besteht aber keine Gefahr einer Beschädigung des Bügelverschlusses während der Implantation und auch danach. Und schließlich ist die erfindungsgemäße Konstruktion ganz erheblich unkomplizierter und damit preisgünstiger als die des nächstkommenden Standes der Technik gemäß US-A 4,292,693.

Durch die klar definierbare Ausbuchtung am Draht und den definierten Durchmesser am Aufnahmeteil, sowie der klar definierten Geometrie des U-Teils, kann die Kraft, die aufzuwenden ist, um das U-Teil elastisch zu öffnen, klar vorhergesagt werden. Damit wird das System im Vergleich zum anderen System in Bezug auf die Anwendung viel sicherer.

Weiter können Geometrie und Maße des Bügels so gestaltet werden, dass immer ein leichter Druck auf dem Ambossfortsatz vorhanden bleibt, was den Vorteil hat, dass kein Spiel am Bügel vorhanden ist. Denkt man dabei an das Risiko des ungewollten Abtauchens der Prothese ins Innenohr, so wird schnell klar, dass der Bügel und seine Selbsthaltekraft dies sicher verhindern wird.

Besonders bevorzugt sind Ausführungsformen der erfindungsgemäßen Gehörknöchelchenprothese, die sich dadurch auszeichnen, dass die Ausbuchtung so weit aus dem Bügelverschluss vorsteht, dass sie während des Verschwenkens des Bügelverschlusses zumindest zeitweilig kraftschlüssig mit einer Andruckkraft an der Außenseite des Aufnahmeteils anliegt beziehungsweise auf diesem mit einer Andruckkraft schleift, und dass der Bügelverschluss aus einem Material aufgebaut ist, welches sich beim Verschwenken unter der Wirkung der Andruckkraft elastisch verformt. Dabei verhindert die Kraft einerseits das Zurückschwenken, andererseits verleiht sie dem Implantat eine Sicherung gegen Abrutschen oder Abtauchen.

Vorteilhaft in der Handhabung und besonders kostengünstig in der Herstellung ist eine Klasse von Ausführungsformen der Erfindung, die dadurch gekennzeichnet sind, dass der Bügelverschluss aus Drahtförmigem Material aufgebaut ist, und dass die Ausbuchtung die Form einer Sicke aufweist und mindestens um die Distanz einer viertel Drahtdicke, vorzugsweise einer halben Drahtdicke, in Richtung auf das Aufnahmeteil vorsteht. Mit dieser Herstellungsart können Kraft und Ausmaße genau definiert werden, um eine eindeutige Reproduzierbarkeit zu erzeugen.

Eine vorteilhafte Weiterbildung dieser Klasse von Ausführungsformen zeichnet sich dadurch aus, dass das Drahtförmige Material, aus welchem der Bügelverschluss aufgebaut ist, einen runden Querschnitt aufweist. Mit dieser Art der Herstellung können sehr kostengünstige Varianten der Erfindung realisiert werden.

Bei alternativen Weiterbildungen weist das Drahtförmige Material, aus welchem der Bügelverschluss aufgebaut ist, einen rechteckigen, vorzugsweise quadratischen Querschnitt auf. Insbesondere kann der Bügelverschluss aus Bandförmigem Material aufgebaut sein.

Ganz besonders bevorzugt sind auch Ausführungsformen der erfindungsgemäßen Gehörknöchelchenprothese, die sich dadurch auskennzeichnen, dass der U-förmige Bügelverschluss zwei im Wesentlichen parallel verlaufende Seitenabschnitte aufweist, die einenends über einen Bügelabschnitt miteinander verbunden und anderenends jeweils in Endzapfen übergehen, welche drehbar in den Aufnahmeteil eingreifen, und dass jeweils an beiden Seitenabschnitten mindestens eine Ausbuchtung angeordnet ist. Dadurch kann die wirkende Kraft quasi über die Geometrie eingestellt werden.

Diese Ausführungsformen lassen sich bei vorteilhaften Weiterbildung noch dadurch verbessern, dass der U-förmige der Bügelverschluss im Bereich des Bügelabschnitts aus der durch die U-Form gebildeten Ebene heraus abgekröpft ist, was zum Vorteil hat, dass teilweise die Auslenkung nach lateral zum Trommelfell hin etwas verkürzt wird, damit die Implantation in Bezug auf die Baugröße vereinfacht wird.

Besonders bevorzugte Ausführungsformen der erfindungsgemäßen Gehörknöchelchenprothese sind dadurch gekennzeichnet, dass der Prothesenkörper mindestens ein Gelenk, insbesondere ein Kugelgelenk aufweist. Dies ist vorteilhaft im Hinblick auf eine besonders hohe postoperative Beweglichkeit der Prothese. Möglich sind auch Weiterbildungen, bei denen eine Vielzahl von aneinander angrenzenden weiteren Drehelementen vorgesehen ist, vorzugsweise eine Kugelgelenkkette. Nachdem die Prothese operativ im Mittelohr platziert wurde und das Trommelfell wieder verschlossen ist, beginnt die so genannte Einheilphase. In dieser Zeit bilden sich Narben und diese verursachen unvorhersehbar Kräfte, welche dazu führen können, die Prothese aus ihrer lokalen Position zu verschieben.

Die erfindungsgemäße Gehörknöchelchenprothese selbst oder Teile davon können aus Titan und/oder aus Gold und/oder aus Tantal und/oder aus Stahl und/oder aus einer Legierung der genannten Metalle hergestellt sein. Insbesondere das Material Titan weist neben seiner Festigkeit und ausgezeichneten Schallleitungseigenschaften bekanntermaßen auch eine hervorragende Biokompatibilität am menschlichen Mittelohr auf.

Vorteilhaft im Hinblick auf die oben erwähnte postoperative Lageanpassung sind Ausführungsformen der Erfindung, bei denen die Gehörknöchelchenprothese oder Teile davon aus einem Material mit Formgedächtnis (=memory effect) und/oder superelastischen Eigenschaften, vorzugsweise aus Nitinol hergestellt sind, was per se beispielsweise aus der WO 02/069850 A1 oder der US 6,554,861 B2 bekannt ist.

Alternativ oder ergänzend können bei weiteren Ausführungsformen Teile der erfindungsgemäßen Gehörknöchelchenprothese aus einem Keramikmaterial hergestellt sein.

Möglich sind aber auch Ausführungsformen der Erfindung, bei denen die gesamte Prothese oder Teile davon aus biokompatiblen Kunststoffen, insbesondere Silikon, Polytetrafluorethylen (PTFE) oder Faserverbundwerkstoffen hergestellt sind. Mit diesen Materialien können post-operative Abstoßungsreaktionen in den meisten Fällen ebenfalls verhindert werden.

Besonders bevorzugt ist eine Ausführungsform der erfindungsgemäßen Gehörknöchelchenprothese, bei der die Massenverteilung der einzelnen Teile der Prothese in Abhängigkeit von einem gewünschten, vorgebbaren Frequenzgang der Schallleitung im Mittelohr berechnet ist. Damit lässt sich ohne großen zusätzlichen technischen Aufwand gewissermaßen ein Tuning der Schallfortpflanzungseigenschaften mittels einer individuellen ausgestalteten Gehörknöchelchenprothese erreichen. Die gezielte Frequenzanpassung zur verbesserten Schallleitung im Mittelohr ist beispielsweise in der EP 1 706 071 B1 oder in der US 7,871,439 B2 beschrieben.

Ein solches "mechanisches Tuning" kann bei speziellen Ausführungsformen beispielsweise dadurch erzielt werden, dass mindestens eine zusätzliche Masse in Abhängigkeit von einem gewünschten, vorgebbaren Frequenzgang der Schallleitung im Mittelohr an einem Teil der Gehörknöchelchenkette bzw. der Prothese befestigt ist. Bei vorteilhaften Weiterbildungen dieser Ausführungsformen ist die zusätzliche Masse mittels eines Clips an einem Teil der Gehörknöchelchenkette oder der Prothese befestigt. Außerdem können die zusätzliche Masse und/oder der Clip ebenfalls mit einer biologisch aktiven Beschichtung überzogen sein.

Weitere Merkmale und Vorteile der Erfindung ergeben sich aus der nachfolgenden detaillierten Beschreibung von Ausführungsbeispielen der Erfindung anhand der Figuren der Zeichnung, die erfindungswesentliche Einzelheiten zeigt, sowie aus den Ansprüchen. Die einzelnen Merkmale können jeweils einzeln für sich oder zu mehreren in beliebigen Kombinationen bei Varianten der Erfindung verwirklicht sein.

In der schematischen Zeichnung sind Ausführungsbeispiele der Erfindung dargestellt, welche in der nachfolgenden Beschreibung näher erläutert werden.

Es zeigen:
- Fig. 1: eine schematische räumliche Darstellung einer ersten Ausführungsform der erfindungsgemäßen Gehörknöchelchenprothese in ihrer Einbausituation nach der Implantation mit einem über den freien Endabschnitt des Ambossfortsatzes verschwenkten Bügelverschluss aus RundDraht sowie einem als Piston ausgestaltetem zweiten Ankoppelelement zum Eingriff durch eine perforierte Steigbügel-Fußplatte hindurch ins Innenohr;
- Fig. 2: eine Ausführungsform ähnlich wie in Fig. 1;
- Fig. 3: eine Ausführungsform wie in Fig. 2, aber mit einem Bügelverschluss aus Draht mit viereckigem Querschnitt sowie mit Bohrungen im Bügel und nur leicht nach innen vorstehender, auf das Aufnahmeteil gerichteter Ausbuchtung;
- Fig. 4: eine Ausführungsform wie in Fig. 3, aber ohne Bohrungen im Bügel, dafür mit Sickenförmig gestalteter Ausbuchtung;
- Fig. 5: eine Ausführungsform wie in Fig. 4, aber mit deutlich tieferer Sicke, die etwa um die Distanz einer halben Drahtdicke in Richtung auf das Aufnahmeteil vorsteht ; und
- Fig. 6: eine Ausführungsform wie in Fig. 1, aber mit einem auf eine künstliche Steigbügel-Fußplatte aufgesetzten zweiten Befestigungselement.

Die in den Figuren der Zeichnung schematisch dargestellten, im Detail unterschiedlich gestalteten Ausführungsformen der erfindungsgemäßen Gehörknöchelchenprothese 10; 20; 30; 40; 50; 60 weisen jeweils einen schaftförmigen länglichen, Schall übertragenden Prothesenkörper 13; 23; 33; 43; 53; 63 auf, welcher am einen Ende jeweils ein erstes Ankoppelelement 11; 21; 31; 41; 51; 61 trägt, das zur mechanischen Verbindung mit dem Ambossfortsatz A gestaltet ist und einen starr mit dem Prothesenkörper 13; 23; 33; 43; 53; 63 verbundenen Aufnahmeteil 11a; 21a; 31a; 41a; 51a; 61a zur Aufnahme des freien Endabschnitts des Ambossfortsatzes A sowie einen im Wesentlichen U-förmig gebogenen Bügelverschluss 11b; 21b; 31b; 41b; 51b; 61b aufweist, welcher drehbar im Aufnahmeteil 11a; 21a; 31a; 41a; 51a; 61a gelagert ist und zur Befestigung des Ambossfortsatzes A über den im Aufnahmeteil 11a; 21a; 31a; 41a; 51a; 61a aufgenommenen freien Endabschnitt des Ambossfortsatzes A geschwenkt werden kann. Am anderen Ende des Prothesenkörpers 13; 23; 33; 43; 53; 63 sitzt ein zweites Ankoppelelement 12; 22; 32; 42; 52; 62, welches in vielfältigen geometrischen Formen zur mechanischen Verbindung mit einem weiteren Glied oder Teilen eines Gliedes der Gehörknöchelchenkette oder als Piston zum direkten Eintauchen ins Innenohr gestaltet sein kann.

Erfindungsgemäß ist die Gehörknöchelchenprothese 10; 20; 30; 40; 50; 60 dadurch gekennzeichnet, dass der Bügelverschluss 11b; 21b; 31b; 41b; 51b; 61b mindestens eine Ausbuchtung 111; 211; 311; 411; 511; 611',611" aufweist, die auf das Aufnahmeteil 11a; 21a; 31a; 41a; 51a; 61a gerichtet ist und so weit aus dem Bügelverschluss 11b; 21b; 31b; 41b; 51b; 61b vorsteht, dass sie beim Verschwenken des Bügelverschlusses 11b; 21b; 31b; 41b; 51b; 61b eine Außenfläche des Aufnahmeteils 11a; 21a; 31a; 41a; 51a; 61a berührt.

Die Ausbuchtung 111; 211; 311; 411; 511; 611',611" steht so weit aus dem Bügelverschluss 11b; 21b; 31b; 41b; 51b; 61b vor, dass sie während des Verschwenkens des Bügelverschlusses 11b; 21b; 31b; 41b; 51b; 61b zumindest zeitweilig kraftschlüssig mit einer Andruckkraft an der Außenseite des Aufnahmeteils 11a; 21a; 31a; 41a; 51a; 61a anliegt beziehungsweise auf diesem mit einer Andruckkraft schleift, und dass der Bügelverschluss 11b; 21b; 31b; 41b; 51b; 61b aus einem Material aufgebaut ist, welches sich beim Verschwenken unter der Wirkung der Andruckkraft elastisch verformt.

Der Bügelverschluss 11b; 21b; 31b; 41b; 51b; 61b ist aus Drahtförmigem Material aufgebaut, wobei die Ausbuchtung 111; 211; 311; 411; 511; 611',611" die Form einer Sicke aufweist und mindestens um die Distanz einer viertel Drahtdicke, vorzugsweise einer halben Drahtdicke in Richtung auf das Aufnahmeteil 11a; 21a; 31a; 41a; 51a; 61a vorsteht.

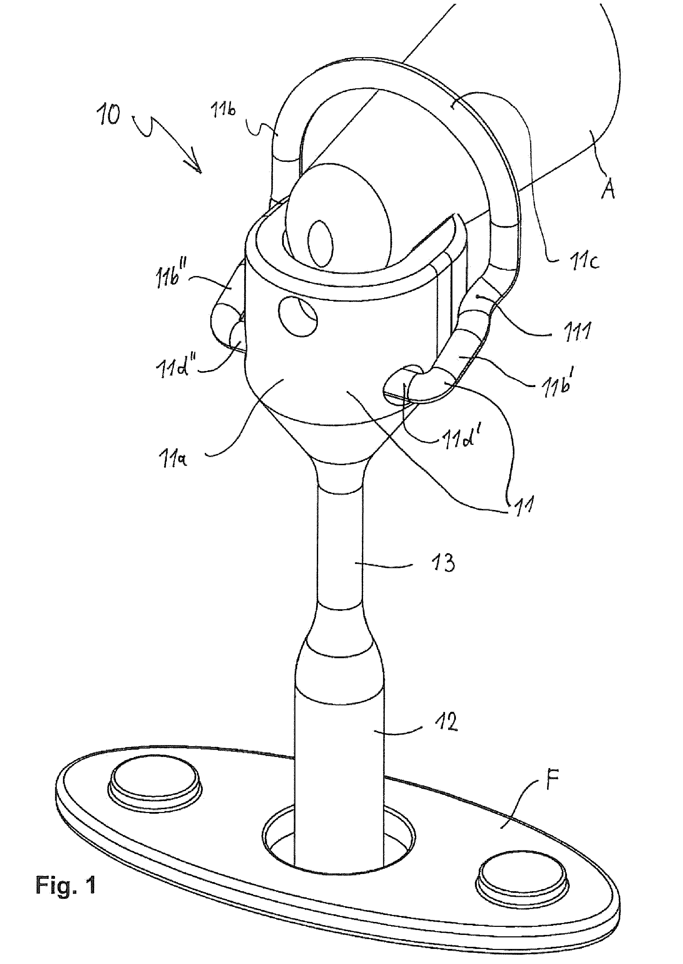

In Fig. 1 greift ein als Piston ausgestaltetes zweites Ankoppelelement 12 durch eine - stilisiert mit den beiden Ansätzen des ehemaligen Steigbügels dargestellte - perforierte Steigbügel-Fußplatte F hindurch ins Innenohr.

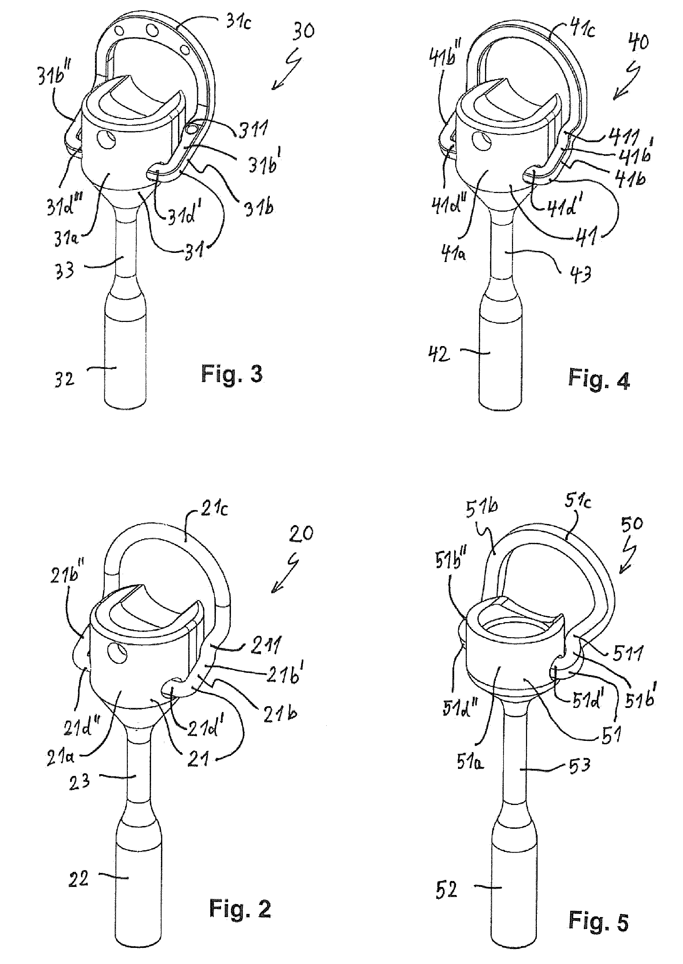

Bei den in den Figuren 1, 2 und 6 gezeigten Ausführungsformen der erfindungsgemäßen Gehörknöchelchenprothese 10; 20; 60 ist das Drahtförmige Material, aus welchem der Bügelverschluss 11b; 21b; 61b aufgebaut und weist einen runden Querschnitt auf. Bei den Ausführungsformen gemäß Figuren 3 bis 5 hat der Draht des Bügelverschlusses 31b; 41b; 51b einen rechteckigen, vorzugsweise quadratischen Querschnitt.

Der U-förmige Bügelverschluss 11b; 21b; 31b; 41b; 51b; 61b weist vorzugsweise zwei im Wesentlichen parallel verlaufende Seitenabschnitte 11b',11b"; 21b',21b"; 31b',31b"; 41b',41b"; 51b',51b"; 61b',61b" auf, die einenends über einen Bügelabschnitt 11c; 21c; 31c; 41c; 51c; 61c miteinander verbunden und anderenends jeweils in Endzapfen 11d',11d"; 21d',21d"; 31d',31d"; 41d',41d"; 51d',51d"; 61d',61d" übergehen, welche drehbar in den Aufnahmeteil 11a; 21a; 31a; 41a; 51a; 61a eingreifen.

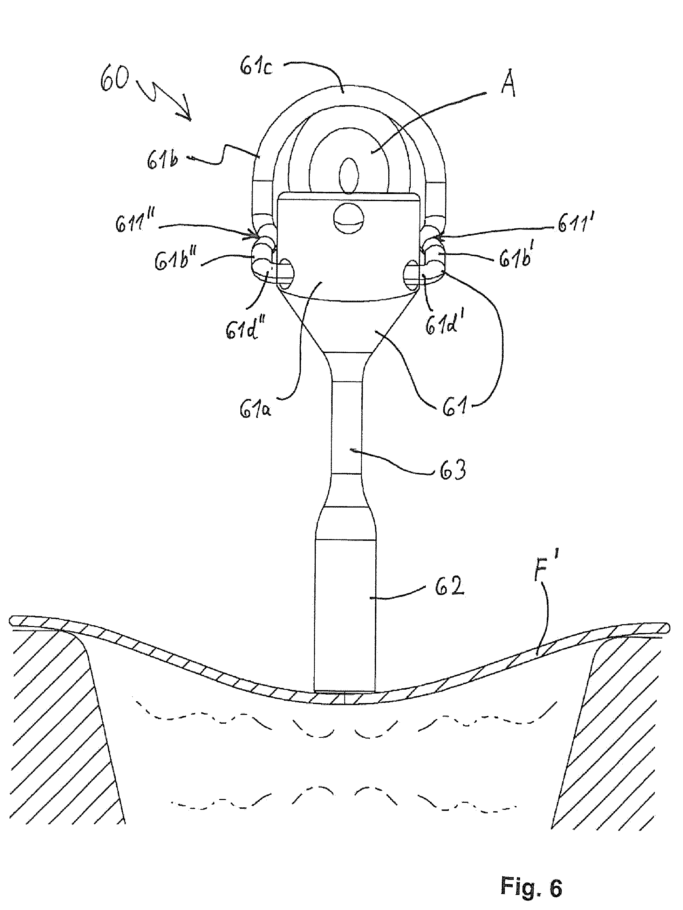

Wie insbesondere aus Fig. 6 deutlich wird, kann jeweils an beiden Seitenabschnitten 61b',61b" mindestens eine Ausbuchtung 611',611" angeordnet sein. Das zweite Ankoppelelement 62 sitzt hier auf einer künstlich - meist aus körpereigenem Gewebe des Patienten - hergestellten Steigbügel-Fußplatte P auf.

Vorzugsweise ist der U-förmige der Bügelverschluss 11b; 21b; 31b; 41b; 51b; 61b im Bereich des Bügelabschnitts 11c; 21c; 31c; 41c; 51c; 61c aus der durch die beiden Schenkel der U-Form gebildeten Ebene heraus abgekröpft.

Der der Prothesenkörper kann - bei in der Zeichnung nicht dargestellten Ausführungsformen der Erfindung - mindestens ein Gelenk, insbesondere ein Kugelgelenk aufweisen.

Die Massenverteilung der einzelnen Teile einer erfindungsgemäßen Gehörknöchelchenprothese kann in Abhängigkeit von einem gewünschten, vorgebbaren Frequenzgang der Schallleitung im Mittelohr derart berechnet sein, dass ein individuelles akustisches Tuning der Schallleitungs-Eigenschaften ermöglicht wird. Dies kann auch - bei in der Zeichnung nicht dargestellten Ausführungsformen der Erfindung - durch an die Gehörknöchelchenprothese anclipsbare "Trimm-Massen" erreicht werden.

## Patentansprüche

1. Gehörknöchelchenprothese (10; 20; 30; 40; 50; 60), die zum Ersatz oder Überbrücken mindestens eines Elements der menschlichen Gehörknöchelchenkette ausgebildet ist, mit einem länglichen, Schall übertragenden Prothesenkörper (13; 23; 33; 43; 53; 63), welcher an seinem einen Ende ein erstes Ankoppelelement (11; 21; 31; 41; 51; 61) aufweist, das zur mechanischen Verbindung mit dem Ambossfortsatz (A) gestaltet ist und einen starr mit dem Prothesenkörper (13; 23; 33; 43; 53; 63) verbundenen Aufnahmeteil (11a; 21a; 31a; 41a; 51a; 61a) zur Aufnahme des freien Endabschnitts des Ambossfortsatzes (A) sowie einen U-förmig gebogenen Bügelverschluss (11b; 21b; 31b; 41b; 51b; 61b) aufweist, welcher drehbar im Aufnahmeteil (11a; 21a; 31a; 41a; 51a; 61a) gelagert ist und zur Befestigung des Ambossfortsatzes (A) über den im Aufnahmeteil (11a; 21a; 31a; 41a; 51a; 61a) aufgenommenen freien Endabschnitt des Ambossfortsatzes (A) geschwenkt werden kann, wobei der Prothesenkörper (13; 23; 33; 43; 53; 63) an seinem anderen Ende ein zweites Ankoppelelement (12; 22; 32; 42; 52; 62) zur mechanischen Verbindung mit einem weiteren Glied oder Teilen eines Gliedes der Gehörknöchelchenkette oder direkt mit dem Innenohr aufweist, **dadurch gekennzeichnet,**
**dass** der Bügelverschluss (11b; 21b; 31b; 41b; 51b; 61b) mindestens eine Ausbuchtung (111; 211; 311; 411; 511; 611',611") aufweist, die auf das Aufnahmeteil (11a; 21a; 31a; 41a; 51a; 61a) gerichtet ist und so weit aus dem Bügelverschluss (11b; 21b; 31b; 41b; 51b; 61b) vorsteht, dass sie beim Verschwenken des Bügelverschlusses (11b; 21b; 31b; 41b; 51b; 61b) eine Außenfläche des Aufnahmeteils (11a; 21a; 31a; 41a; 51a; 61a) berührt.

2. Gehörknöchelchenprothese nach Anspruch 1, **dadurch gekennzeichnet, dass** die Ausbuchtung (111; 211; 311; 411; 511; 611',611") so weit aus dem Bügelverschluss (11b; 21b; 31b; 41b; 51b; 61b) vorsteht, dass sie während des Verschwenkens des Bügelverschlusses (11b; 21b; 31b; 41b; 51b; 61b) zumindest zeitweilig kraftschlüssig mit einer Andruckkraft an der Außenseite des Aufnahmeteils (11a; 21a; 31a; 41a; 51a; 61a) anliegt beziehungsweise auf diesem mit einer Andruckkraft schleift, und dass der Bügelverschluss (11b; 21b; 31b; 41b; 51b; 61b) aus einem Material aufgebaut ist, welches sich beim Verschwenken unter der Wirkung der Andruckkraft elastisch verformt.

3. Gehörknöchelchenprothese nach einem der vorhergehenden Ansprüche, **dadurch gekennzeichnet, dass** der Bügelverschluss (11b; 21b; 31b; 41b; 51b; 61b) aus Drahtförmigem Material aufgebaut ist, und dass die Ausbuchtung (111; 211; 311; 411; 511; 611',611") die Form einer Sicke aufweist und mindestens um die Distanz einer viertel Drahtdicke in Richtung auf das Aufnahmeteil (11a; 21a; 31a; 41a; 51a; 61a) vorsteht.

4. Gehörknöchelchenprothese nach Anspruch 3 **dadurch gekennzeichnet, dass** die Ausbuchtung (111; 211; 311; 411; 511; 611',611") mindestens um die Distanz einer halben Drahtdicke in Richtung auf das Aufnahmeteil (11a; 21a; 31a; 41a; 51a; 61a) vorsteht.

5. Gehörknöchelchenprothese nach Anspruch 3 oder 4, **dadurch gekennzeichnet, dass** das Drahtförmige Material, aus welchem der Bügelverschluss (11b; 21b; 61b) aufgebaut ist, einen runden Querschnitt aufweist.

6. Gehörknöchelchenprothese nach Anspruch 3 oder 4, **dadurch gekennzeichnet, dass** das Drahtförmige Material, aus welchem der Bügelverschluss (31b; 41b; 51b) aufgebaut ist, einen rechteckigen Querschnitt aufweist.

7. Gehörknöchelchenprothese nach einem der vorhergehenden Ansprüche, **dadurch gekennzeichnet, dass** der U-förmige Bügelverschluss (11b; 21b; 31b; 41b; 51b; 61b) zwei parallel verlaufende Seitenabschnitte (11b',11b"; 21b',21b"; 31b',31b"; 41b',41b"; 51b',51b"; 61b',61b") aufweist, die einenends über einen Bügelabschnitt (11c; 21c; 31c; 41c; 51c; 61c) miteinander verbunden und anderenends jeweils in Endzapfen (11d',11d"; 21d',21d"; 31d',31d"; 41d',41d"; 51d',51d"; 61d',61d") übergehen, welche drehbar in den Aufnahmeteil (11a; 21a; 31a; 41a; 51a; 61a) eingreifen, und dass jeweils an beiden Seitenabschnitten (11b',11b"; 21b',21b"; 31b',31b"; 41b',41b"; 51b',51b"; 61b',61b") mindestens eine Ausbuchtung (111; 211; 311; 411; 511; 611',611") angeordnet ist.

8. Gehörknöchelchenprothese nach Anspruch 7, **dadurch gekennzeichnet, dass** der U-förmige der Bügelverschluss (11b; 21b; 31b; 41b; 51b; 61b) im Bereich des Bügelabschnitts (11c; 21c; 31c; 41c; 51c; 61c) aus der durch die U-Form gebildeten Ebene heraus abgekröpft ist.

9. Gehörknöchelchenprothese nach einem der vorhergehenden Ansprüche, **dadurch gekennzeichnet, dass** die Gehörknöchelchenprothese (10; 20; 30; 40; 50; 60) ganz oder teilweise aus einem Material mit Formgedächtnis und/oder mit superelastischen Eigenschaften hergestellt ist.

10. Gehörknöchelchenprothese nach einem der vorhergehenden Ansprüche, **dadurch gekennzeichnet, dass** der Prothesenkörper mindestens ein Gelenk aufweist.

## Claims

1. Auditory ossicle prosthesis (10; 20; 30; 40; 50; 60) which is configured for replacing or bridging at least one element of the human auditory ossicle chain, having an elongate sound-transmitting prosthesis body (13; 23; 33; 43; 53; 63) which comprises at the first end thereof a first attachment element (11; 21; 31; 41; 51; 61) which is configured for mechanical connection to the anvil projection (A) and a receptacle part (11a; 21a; 31a; 41a; 51a; 61a) which is rigidly connected to the prosthesis body (13; 23; 33; 43; 53; 63), for receiving the free end section of the anvil projection (A) and a U-shaped curved clip lock (11b; 21b; 31b; 41b; 51b; 61b) which is rotatably mounted in the receptacle part (11a; 21a; 31a; 41a; 51a; 61a) and for fastening the anvil projection (A) can be pivoted over the free end section of the anvil projection (A) which is received in the receptacle part (11a; 21a; 31a; 41a; 51a; 61a), wherein the prosthesis body (13; 23; 33; 43; 53; 63) has a second attachment element (12; 22; 32; 42; 52; 62) at the other end thereof for mechanical connection to a further member or parts of a member of the auditory ossicle chain or directly to the inner ear,
**characterised in that** the clip lock (11b; 21b; 31b; 41b; 51b; 61b) has at least one bulge (111; 211; 311; 411; 511; 611', 611'') which is directed toward the receptacle part (11a; 21a; 31a; 41a; 51a; 61a) and projects far enough from the clip lock (11b; 21b; 31b; 41b; 51b; 61b) so that, when the clip lock (11b; 21b; 31b; 41b; 51b; 61b) is pivoted, said bulge touches an outer surface of the receptacle part (11a; 21a; 31a; 41a; 51a; 61a).

2. Auditory ossicle prosthesis according to claim 1, **characterised in that** the bulge (111; 211; 311; 411; 511; 611', 611'') projects far enough from the clip lock (11b; 21b; 31b; 41b; 51b; 61b) so that, during the pivoting of the clip lock (11b; 21b; 31b; 41b; 51b; 61b), said bulge at least temporarily lies in a frictionally engaged manner against the outside of the receptacle part (11a; 21a; 31a; 41a; 51a; 61a) with a pressing force or slides thereagainst with a pressing force and **in that** the clip lock (11b; 21b; 31b; 41b; 51b; 61b) is made of a material which deforms elastically under the effect of the pressing force while pivoting.

3. Auditory ossicle prosthesis according to one of the preceding claims, **characterised in that** the clip lock (11b; 21b; 31b; 41b; 51b; 61b) is made of wire-shaped material and **in that** the bulge (111; 211; 311; 411; 511; 611', 611'') has the form of a bead and projects by at least the distance of one quarter wire thickness in the direction toward the receptacle part (11a; 21a; 31a; 41a; 51a; 61a).

4. Auditory ossicle prosthesis according to claim 3, **characterised in that** the bulge (111; 211; 311; 411; 511; 611', 611'') projects by at least the distance of one half wire thickness in the direction toward the receptacle part (11a; 21a; 31a; 41a; 51a; 61a).

5. Auditory ossicle prosthesis according to claim 3 or 4, **characterised in that** the wire-shaped material of which the clip lock (11b; 21b; 61b) is made has a round cross-section.

6. Auditory ossicle prosthesis according to claim 3 or 4, **characterised in that** the wire-shaped material of which the clip lock (31b; 41b; 51b) is made has a rectangular cross-section.

7. Auditory ossicle prosthesis according to one of the preceding claims, **characterised in that** the U-shaped clip lock (11b; 21b; 31b; 41b; 51b; 61b) comprises two parallel extending side sections (11b', 11b"; 21b', 21b"; 31b', 31b"; 41b', 41b"; 51b', 51b"; 61b', 61b") which are connected to one another at one end by means of a hoop portion (11c; 21c; 31c; 41c; 51c; 61c) and at the other end respectively transition into end posts (11d', 11d"; 21d', 21d"; 31d', 31d''; 41d', 41d"; 51d', 51d''; 61d', 61d") which engage rotatably in the receptacle part (11a; 21a; 31a; 41a; 51a; 61a) and **in that** at least one bulge (111; 211; 311; 411; 511; 611', 611'') is arranged on each of the two side sections (11b', 11b"; 21b', 21b''; 31b', 31b''; 41b', 41b"; 51b', 51b"; 61b', 61b'').

8. Auditory ossicle prosthesis according to claim 7, **characterised in that** the U-shaped clip lock (11b; 21b; 31b; 41b; 51b; 61b) is bent over in the region of the hoop portion (11c; 21c; 31c; 41c; 51c; 61c) out of the plane formed by the U-shape.

9. Auditory ossicle prosthesis according to one of the preceding claims, **characterised in that** the auditory ossicle prosthesis (10; 20; 30; 40; 50; 60) is made wholly or partially of a material with shape memory and/or with superelastic properties.

10. Auditory ossicle prosthesis according to one of the preceding claims, **characterised in that** the prosthesis body comprises at least one joint.

## Revendications

1. Prothèse pour osselets de l'oreille (10 ; 20 ; 30 ; 40 ; 50 ; 60), qui est constituée pour remplacer ou combler au moins un élément de la chaîne des osselets humaine, avec un corps de prothèse (13 ; 23 ; 33 ; 43 ; 53 ; 63) oblong transmettant le son qui présente à une extrémité un premier élément d'accouplement (11 ; 21 ; 31 ; 41 ; 51 ; 61) qui est conçu pour lé raccordement mécanique au prolongement de l'enclume (A) et qui présente une partie de réception (11a ; 21a ; 31a ; 41a ; 51a ; 61a) raccordée de façon rigide au corps de prothèse (13 ; 23 ; 33 ; 43 ; 53 ; 63) pour la réception du tronçon d'extrémité libre du prolongement de l'enclume (A) ainsi qu'une fermeture d'étrier (11 b ; 21 b ; 31b ; 41 b ; 51b ; 61b) courbée en U qui est supportée en rotation dans la partie de réception (11a ; 21a ; 31a ; 41a ; 51a ; 61a) et qui peut être amenée à pivoter pour la fixation du prolongement de l'enclume (A) par le biais du tronçon d'extrémité libre du prolongement de l'enclume (A) reçu dans la partie de réception (11a ; 21 a ; 31 a ; 41 a ; 51 a ; 61a), dans laquelle le corps de prothèse (13 ; 23 ; 33 ; 43 ; 53 ; 63) présente à son autre extrémité un deuxième élément d'accouplement (12 ; 22 ; 32 ; 42 ; 52 ; 62) pour le raccordement mécanique à un autre organe ou à des parties d'un organe de la chaîne des osselets ou directement à l'oreille interne, **caractérisée en ce que**
la fermeture d'étrier (11b ; 21b ; 31b ; 41b ; 51b ; 61b) présente au moins un renflement (111 ; 211 ; 311 ; 411 ; 511 ; 611', 611") qui est dirigé vers la partie de réception (11a ; 21a ; 31a ; 41a ; 51a ; 61 a) et qui fait saillie à partir de la fermeture d'étrier (11 b ; 21 b ; 31b ; 41b ; 51b ; 61b) suffisamment loin pour toucher une surface extérieure de la partie de réception (11a ; 21a ; 31a ; 41 a ; 51 a ; 61 a) lors du pivotement de la fermeture d'étrier (11b ; 21b ; 31b ; 41b ; 51b ; 61b).

2. Prothèse pour osselets de l'oreille selon la revendication 1, **caractérisée en ce que** le renflement (111 ; 211 ; 311 ; 411 ; 511 ; 611', 611") fait saillie à partir de la fermeture d'étrier (11b; 21b; 31b; 41b; 51b; 61b) suffisamment loin pour venir appuyer, pendant le pivotement de la fermeture d'étrier (11 b ; 21 b ; 31 b ; 41 b ; 51 b ; 61 b), au moins par intermittence en liaison de force, avec une force de pression, contre le côté extérieur de la partie de réception (11 a ; 21 a ; 31 a ; 41 a ; 51 a ; 61 a) ou respectivement pour frotter contre celle-ci avec une force de pression, et **en ce que** la fermeture d'étrier (11 b ; 21 b ; 31 b ; 41 b ; 51 b ; 61 b) est réalisée dans un matériau qui se déforme élastiquement lors du pivotement sous l'action de la force de pression.

3. Prothèse pour osselets de l'oreille selon l'une des revendications précédentes, **caractérisée en ce que** la fermeture d'étrier (11 b ; 21 b ; 31 b ; 41 b ; 51 b ; 61 b) est réalisée dans un matériau en forme de fil métallique, et **en ce que** le renflement (111 ; 211 ; 311 ; 411 ; 511 ; 611', 611 ") présente la forme d'une nervure et fait saillie, sur au moins la distance d'un quart d'épaisseur de fil métallique, en direction de la partie de réception (11a ; 21a ; 31a ; 441a ; 51a ; 61a).

4. Prothèse pour osselets de l'oreille selon la revendication 3, **caractérisée en ce que** le renflement (111 ; 211 ; 311 ; 411 ; 511 ; 611', 611 ") fait saillie, sur au moins la distance d'une demi-épaisseur de fil métallique, en direction de la partie de réception (11a ; 21a ; 31 a ; 41a ; 51a ; 61a).

5. Prothèse pour osselets de l'oreille selon la revendication 3 ou 4, **caractérisée en ce que** le matériau en forme de fil métallique dont est constituée la fermeture d'étrier (11b ; 21b ; 61 b) présente une section transversale ronde.

6. Prothèse pour osselets de l'oreille selon la revendication 3 ou 4, **caractérisée en ce que** le matériau en forme de fil métallique dont est constituée la fermeture d'étrier (31b ; 41b ; 51 b) présente une section transversale rectangulaire.

7. Prothèse pour osselets de l'oreille selon l'une des revendications précédentes, **caractérisée en ce que** la fermeture d'étrier (11b ; 21 b ; 31 b ; 41 b ; 51 b ; 61 b) en U présente deux tronçons latéraux (11b', 11b" ; 21b', 21b"; 31b', 31 b" ; 41b', 41 b" ; 51 b', 51 b" ; 61 b', 61 b") parallèles qui sont raccordés l'un à l'autre à une extrémité par le biais d'un tronçon d'étrier (11c; 21 c ; 31 c ; 41c; 51c; 61 c) et qui se transforment à l'autre extrémité respectivement en tourillons d'extrémité (11d', 11d" ; 21d', 21d" ; 31d', 31d" ; 41d', 41d" ; 51 d', 51d" ; 61 d', 61d") qui engrènent de façon rotative dans la partie de réception (11a ; 21a ; 31a ; 41a ; 51 a ; 61a), et **en ce qu'**au moins un renflement (111 ; 211 ; 311 ; 411 ; 511 ; 611', 611") est disposé respectivement sur les deux tronçons latéraux (11b', 11b" ; 21b', 21b" ; 31b', 31b' '; 41b', 41b" ; 51b', 51b" ; 61b', 61b").

8. Prothèse pour osselets de l'oreille selon la revendication 7, **caractérisée en ce que**, dans la zone du tronçon d'étrier (11 c ; 21 c ; 31 c ; 41 c ; 51 c ; 61c), la fermeture d'étrier (11 b ; 21b ; 31 b ; 41 b ; 51 b ; 61b) en U est coudée à partir du plan formé par la forme en U.

9. Prothèse pour osselets de l'oreille selon l'une des revendications précédentes, **caractérisée en ce que** la prothèse pour osselets de l'oreille (10 ; 20 ; 30 ; 40 ; 50 ; 60) est fabriquée en totalité ou en partie dans un matériau à mémoire de forme et/ou ayant des caractéristiques super élastiques.

10. Prothèse pour osselets de l'oreille selon l'une des revendications précédentes, **caractérisée en ce que** le corps de prothèse présente au moins une articulation.
